# EUROPEAN PATENT APPLICATION

(11) **EP 4 477 192 A1**
(43) Date of publication of application: **18.12.2024**
(21) Application number: 23766808.2
(22) Date of filing: 06.03.2023
(51) Int. Cl.: A61F 5/44, A61F 13/495, A61F 13/532

(54) **WEARABLE ARTICLE**

(30) Priority: 07.03.2022 JP 2022034357
(71) Applicant: Zuiko Corporation, Ibaraki-shi Osaka 5670082 (JP)
(72) Inventor: KOSHIJIMA, Miwa, Ibaraki-shi, Osaka 567-0082 (JP)
(74) Representative: Hasegawa, Kan
(86) International application number: PCT/JP2023/008413
(87) International publication number: WO 2023/171632

(57) **Abstract**

A diaper (6) is to be nipped by an inner cup and an outer cup to be fitted to an outside of the inner cup for holding the inner cup to the wearer in a state that the inner cup opposes an inguinal region of the wearer. The diaper (6) includes a nipped sheet part (12) to be nipped by the inner cup (20) and the outer cup (30), and having unit passage holes (8a), (73a), and a first absorption part (11) arranged outside of the nipped sheet part (12) in a plan view. The nipped sheet part (12) includes a thin sheet part (14) that is provided in an outer peripheral portion of the nipped sheet part (12) and is thinner than the first absorption part (11), and a second absorption part (13) that is provided inside the thin sheet part (14) in the plan view.

## Description

### Technical Field

The present invention relates to a wearable article which is used in an excrement processing apparatus for suctioning and discharging excrement received in an excrement cup, and holds the excrement cup to a wearer.

### Background Art

Excrement processing apparatuses that suction and discharge excrement or urine and feces of a person who needs care are recently seen in caregiving sites and the like. The excrement processing apparatus includes an excrement cup that receives excrement of a wearer, a hose having one end connected to the excrement cup to introduce the excrement therethrough, a processing unit that is connected to the other end of the hose and is configured to store the excrement suctioned under depressurization in the inside of the hose, and a wearable article for holding the excrement cup to the wearer in a supine position in a state that the excrement cup comes into a tight contact with an inguinal region of the wearer.

A disposable diaper disclosed in Patent Literature 1 is known as an example of such wearable article. Specifically, the diaper is attached with an excrement cup by being nipped by an inner cup and an outer cup of the excrement cup. The diaper attached with the excrement cup is then put on the wearer. The excrement cup can be thus held to the wearer in a state that the excrement cup comes into a tight contact with the inguinal region of the wearer.

Specifically, the excrement cup includes the inner cup that faces the inguinal region of the wearer and the outer cup that is fitted to an outside of the inner cup in a state that the diaper is interposed therebetween. The diaper is to be nipped by the inner cup and the outer cup. The inner cup includes a receiving part that receives excrement and a connection tube that extends from the receiving part to an outside and is to be connected to the hose. The outer cup includes a fitting recess part to be fitted to the outside of the inner cup and a through hole part in communication with the fitting recess part for guiding the connection tube of the inner cup fitted in the fitting recess part to the outside of the fitting recess part.

On the other hand, the diaper includes a diaper main body which is made of paper and has a small thickness and a high strength, a cushioning member which has a water-absorbability and is arranged over the diaper main body and is formed in a central portion with a cup window for the excrement cup, and an inner waterproof paper piece arranged in the cup window of the cushioning member. The diaper is attached with the excrement cup by nipping the inner waterproof paper piece by the inner cup and outer cup. The inner waterproof paper piece is formed with an opening passing therethrough in a thickness direction. The connection part of the inner cup and the hose are connected via the opening.

The diaper enables easy attachment and detachment of the excrement cup to and from the diaper with keeping high integrality of the both, and keeping of the inner cup in a proper posture when being worn.

However, the diaper of Patent Literature 1 is liable to cause a leakage of the excrement from the inner cup to a periphery thereof when the wearer moves from the supine position after relieving or there is a large amount of excrement. There is a likelihood that a part of the leaking excrement comes out of the diaper, resulting in contaminating the outer cup or bedclothes.

Specifically, the excrement leaking from the inner cup is designed to be absorbed exclusively by the cushioning member around the inner waterproof paper piece. However, since an outer peripheral edge of the inner waterproof paper piece is provided near an edge of the opening in the inner cup, a part of the leaking excrement is liable to seep between the inner cup and the inner waterproof paper piece due to a posture of the wearer and an amount of the leaking excrement, resulting in leaking to the outer cup and the bedclothes through the opening. The diaper leaves room for improvement in this respect.

### Citation List

### Patent Literature

Patent Literature 1: Japanese Unexamined Patent Publication No. 2019-180452

### Summary of Invention

The present invention has been made in view of the problem described above, and an object thereof is to provide a wearable article that makes it possible to suppress a leakage of excrement leaking from an inner cup while keeping the inner cup in proper holding to the wearer.

A wearable article which has solved the problem described above is a wearable article to be nipped by an inner member including a receiving part for receiving excrement of a wearer and a connection part extending from the receiving part to an outside and to be connected to a processing unit for processing the excrement received in the receiving part, and an outer member including a fitting recess part to be fitted to an outside of the inner member and a through hole in communication with the fitting recess part for allowing the connection part to pass, for holding the inner member to the wearer in a state that the inner member opposes an inguinal region of the wearer, and including a nipped sheet part to be nipped by the inner member and the outer member by the fitting of the inner member in the fitting recess part of the outer member, and having an opening for allowing the connection part to pass, and a first absorption part arranged outside of the nipped sheet part in a plan view for absorbing the excrement leaking from the receiving part, wherein the nipped sheet part includes a thin sheet part that is provided at least in an outer peripheral portion of the nipped sheet part and is thinner than the first absorption part, and a second absorption part that is provided inside the thin sheet part in the plan view.

The wearable article makes it possible to maintain a correct holding state of the inner member by the wearer and suppress a leakage of the excrement leaking from the inner member through an opening of the wearable article to thereby effectively suppress the contamination of the outer member and the bedclothes with the excrement.

### Brief Description of Drawings

FIG. 1 is a partially schematic perspective view of an automatic excrement processing apparatus to which a wearable article is used.
FIG. 2 is an exploded perspective view of an excrement cup.
FIG. 3 is a perspective view of a wearable article.
FIG. 4 is an exploded perspective view of the wearable article.
FIG. 5 is a plan view showing a development of the wearable article.
FIG. 6 is a sectional view taken along a line VI-VI in FIG. 5.
FIG. 7 is a sectional view taken along a line VII-VII in FIG. 5.
FIG. 8 is a schematic sectional view of the wearable article attached with the excrement cup.

### Description of Embodiments

Hereinafter, an embodiment of the present invention will be described with reference to the drawings. It should be noted that the below-described embodiment is a specific example of the present invention, and will not delimit the technical scope of the present invention. Further, a wearable article described in this embodiment is used to an automatic excrement processing apparatus configured to automatically suction and process excrement. However, a wearable article may be appreciated to be used to an excrement processing apparatus in which an excrement suction operation is manually managed. Further, a wearable article described in this embodiment is a disposable diaper in the so-called tape form. However, the present invention may be applied to a disposable diaper in the so-called pants form, a washable diaper capable of repeated use, or a combination of a diaper cover and a disposable water-absorbent sheet, and thus does not limit to a wearable article having a particular form.

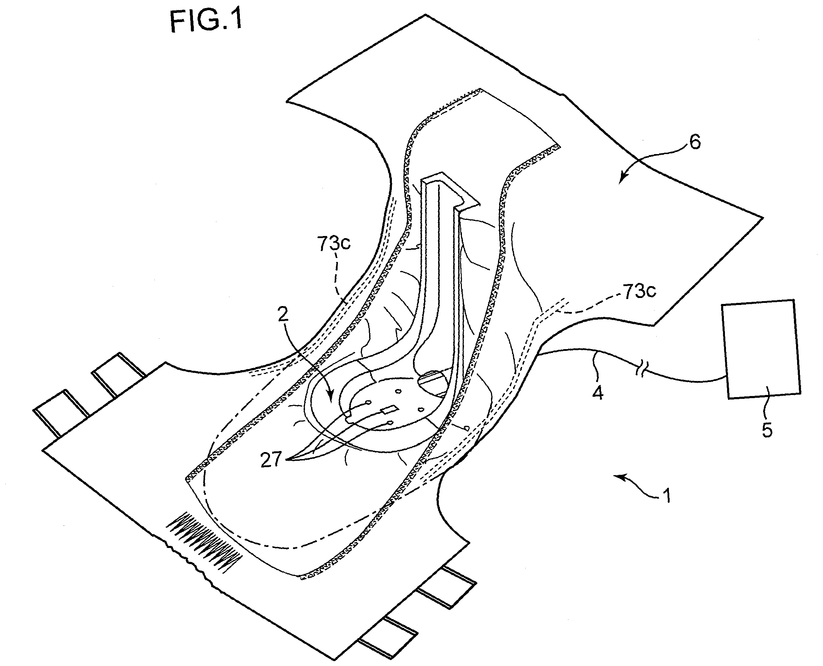

FIG. 1 is a partially schematic perspective view of an automatic excrement processing apparatus to which the wearable article is used. The automatic excrement processing apparatus 1 includes an excrement cup 2 that receives excrement of a wearer (e.g., a person who needs care), a hose 4 having an end connected to the excrement cup 2 to allow the excrement to pass therethrough, a processing unit 5 that is connected to the other end of the hose 4 and stores the excrement suctioned under depressurization in the inside of the hose 4, and a diaper 6 (an example of the wearable article: hereinafter, simply referred to as "diaper") for holding the excrement cup 2 to the wearer in a state that the excrement cup 2 comes into tight contact with an inguinal region of the wearer. In the automatic excrement processing apparatus 1, a below-described excrement sensor 27 disposed in the excrement cup 2 detects receiving of excrement by the excrement cup 2, and the processing unit 5 operates in response to a signal from the sensor 27 to automatically suction the excrement from the excrement cup 2 into the processing unit 5 via the hose 4. Hereinafter, the excrement cup 2 to be attached to the diaper 6 will be specifically described, and then a specific description will be made about the diaper 6.

### Excrement Cup 2

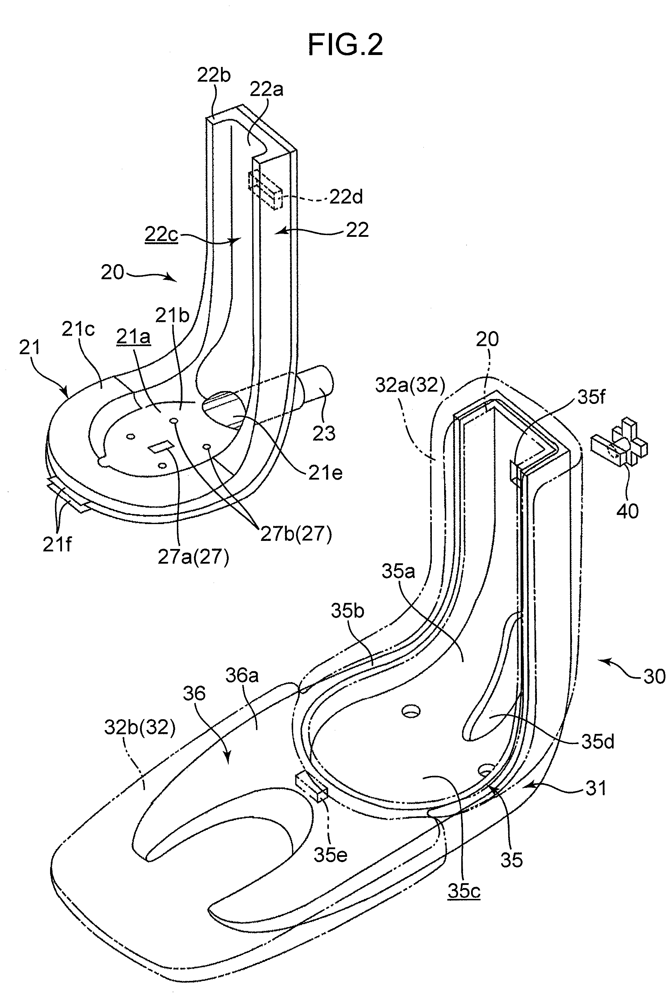

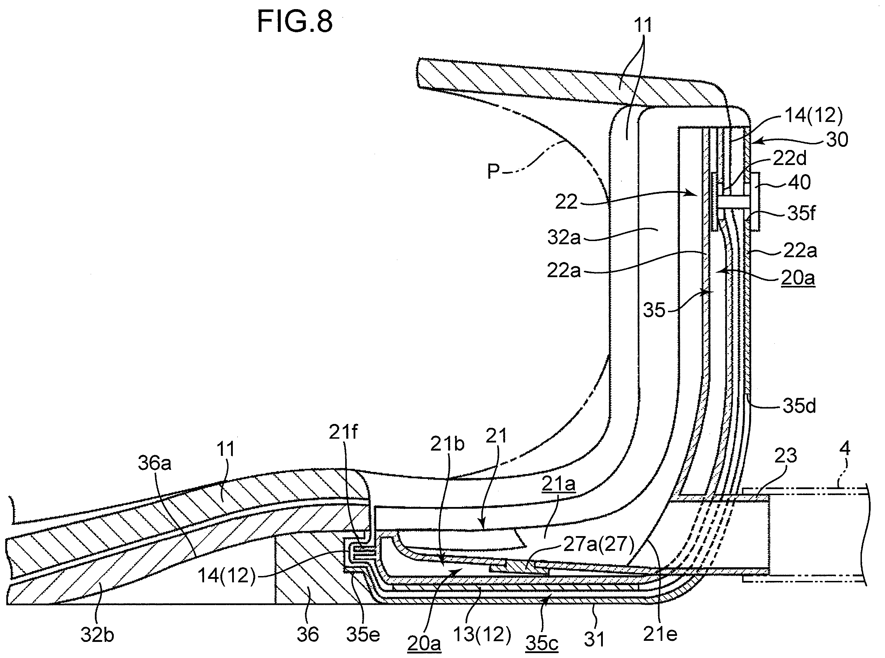

FIG. 2 is an exploded perspective view of the excrement cup 2. When being worn, the excrement cup 2 is considered to be in a state of coming into a tight contact with the inguinal region of the wearer P in the supine position (see FIG. 8). The excrement cup 2 includes an inner cup 20 (an example of an inner member) that opposes the inguinal region of the wearer, an outer cup 30 (an example of an outer member) that is detachably attached to an outside of the inner cup 20 via the diaper 6 interposed therebetween, and a lock pin 40 that connects the inner cup 20 to the outer cup 30. The diaper 6 is attached with the excrement cup 2 by being nipped by the inner cup 20 and the outer cup 30. The lock pin 40 connects the cup 20 to the cup 30, but may be omitted in a case that, for example, an alternative attaching mechanism is provided.

Specifically, with reference to FIG. 2 and FIG. 8, the inner cup 20 substantially has a shape of a ladle in an overall view. Specifically, the inner cup 20 includes a cup main body 21 (an example of a receiving part) that is a lower part of the inner cup 20 in a state where the wearable article is worn by the wearer P in the supine position, an urination guide part 22 that extends upward from a rear end portion of the cup main body 21, and a drain tube part 23 connected to the cup main body 21 below the urination guide part 22. In this embodiment, the cup main body 21 and the urination guide part 22 are formed to have hollows defined by respective inner outer double wall portions, and thus have inner spaces 20a in communication with each other. The inner cup 20 further includes an excrement sensor 27 mounted on the cup main body 21. As described later, the drain tube part 23 is an example of "a connection part" of the present invention, and protrudes rearward from the cup main body 50 to connect the excrement cup 2 with the processing unit 5.

The cup main body 21 includes a bottom wall 21b having an upper surface sloping down rearwardly and a peripheral wall 21c rising upward from an outer peripheral portion of the bottom wall 21b. The cup main body 21 defines a receiving space 21a surrounded by the bottom wall 21b and the peripheral wall 21c. The receiving space 21a temporally accommodates excrement such as feces and urine. As described above, the bottom wall 21b and the peripheral wall 21c are formed to have hollows defined by the respective inner outer double wall portions, and thus have the inner spaces 20a inside the double wall portions.

The excrement sensor 27 is water-tightly attached to the bottom wall 21b in an orientation to the receiving space 21a from the inner space 20a. Specifically, a faecal sensor 27a (an example of the excrement sensor 27) is provided at a substantially central position of the bottom wall 21b, and a plurality of urinal sensors 27b (an example of the excrement sensor 27) is provided around the faecal sensor 27a. The sensors 27a and 27b are electrically connected to the processing unit 5 via a power cord not shown.

The peripheral wall 21c has a rear end portion formed with a discharge opening 21e that faces the receiving space 21a. The discharge opening 21e is connected with one end opening of the drain tube part 23. Excrement in the receiving space 21a is discharged through the discharge opening 21e.

The peripheral wall 21c includes a front end portion formed with an inner side engaging portion 21f (an example of an engaging portion). The inner side engaging portion 21f serves to engage the inner cup 20 with the outer cup 30, and establishes a detachable attachment of the inner cup 20 to the outer cup 30 in cooperation with the lock pin 40 in a state where the inner side engaging portion 21f is engaged with an outer side engaging recess 35e to be described later. The inner side engaging portion 21f includes a protrusion part protruding frontward, and specifically, includes a pair of upper and lower tongues in the present embodiment.

The urination guide part 22 guides urine relieved from the wearer to the receiving space 21a of the cup main body 21. The urination guide part 22 has a shape of a gutter extending upward. Specifically, the urination guide part 22 includes a rear wall 22a extending upward and side walls 22b projecting from both left and right end portions of the rear wall 22a. The rear wall 22a and the side walls 22b define a guiding groove 22c for guiding urine. The guiding groove 22c joins the receiving space 21a.

The rear wall 22a is formed with an inner space 20a defined by a front rear double wall. The rear wall portion of the rear wall 22a has an upper end portion formed with a pin through hole 22d which allows the lock pin 40 to pass therethrough.

The drain tube part 23 is a tubular member for connecting the discharge opening 21e of the cup main body 21 with the hose 4. In this embodiment, the drain tube part 23 is formed integrally with the cup main body 21. The drain tube part 23 protrudes rearward from the cup main body 21, and has a protruding end positioning behind the outer cup 30, and is to be connected to the hose 4.

Hereinafter, the outer cup 30 will be described with reference to FIG. 2 and FIG. 8.

The outer cup 30 includes an outer base body 31 that is fitted with an outside of the inner cup 20 to allow the inner cup 20 to be attached thereto, and a soft part 32 that is attached to the outer base body 31 and to be interposed between the outer base body 31 and the wearer. The soft part 32 may be appropriately omitted. However, it is noted that the provision of the soft part 32 can make the wearer have a comfortable wearing feeling for the excrement cup 2.

The outer base body 31 includes an inner accommodation part 35 for accommodating the cup main body 21 and the urination guide part 22 of the inner cup 20 and a seat part 36 extending frontward from a front end portion of the inner accommodation part 35.

The inner accommodation part 35 includes an outer wall main body 35a extending along a rear surface of the urination guide part 22 from a lower surface of the cup main body 21 and, and an outer peripheral wall 35b projecting from an outer peripheral portion of the outer wall main body 35a. The inner accommodation part 35 includes a fitting recess part 35c surrounded by the outer wall main body 35a and the outer peripheral wall 35b, and is fitted with the outside of the inner cup 20 in a state where the diaper 6 is interposed between the inner cup 20 and the fitting recess part 35c. The fitting recess part 35c has such a size that the outer peripheral surface of the inner cup 20 is tightly fitted in the fitting recess part 35c but a gap exists between a lower surface of the inner cup 20 and the fitting recess part 35c. The outer peripheral surface of the inner cup 20 means an outer surface of the peripheral wall 21c of the cup main body 21 and respective outer surfaces of the side walls 22b of the urination guide part 22. Further, the lower surface of the inner cup 20 means a lower surface of the bottom wall 21b of the cup main body 21.

The outer wall main body 35a is formed into a curved plate-like-shape extending upward so as to follow from the cup main body 21 to the urination guide part 22 of the inner cup 20. The outer wall main body 35a includes an upper portion formed with a pin through hole 35f allowing the lock pin 40 to pass therethrough. Further, the outer wall main body 35a includes an outer through hole 35d (an example of a through hole) allowing the drain tube part 23 of the inner cup 20 to pass therethrough, and the outer through hole 35d is provided below the pin through hole 35f.

In this embodiment, the outer peripheral wall 35b has a height that makes the outer peripheral wall 35b protrude beyond the inner cup 20 toward the wearer. Therefore, the inner cup 20 is fitted in the fitting recess part 35c of the outer cup 30, and is kept from coming into direct contact with the wearer. The outer peripheral wall 35b includes a front end portion formed with the outer side engaging recess 35e (an example of the engaging portion) in which the inner side engaging portion 21f is fitted to thereby engage with the outer side engaging recess 35e. The outer side engaging recess 35e joins the fitting recess part 35c, and has a size which allows the inner side engaging portion 2 1f to be fitted therein with a below-described thin sheet part 14 of the diaper 6 interposed therebetween.

The seat part 36 serves to gradually lift upward the buttocks of the wearer, and has a shape of a flat plate and is formed with a downgauged portion. The seat part 36 in the present embodiment includes an inclined surface 36a rising up as advancing rearward.

The soft part 32 serves to reduce a discomfort of the wearer when coming to the wearer via the diaper 6, and is made of a soft material, e.g., a rubber material or a foamed material, having an elasticity. The soft part 32 includes a first soft part 32a covering a top portion of the outer peripheral wall 35b and a second soft part 32b covering the seat part 36. The soft parts 32a and 32b may be integrally formed or separately formed. A soft part other than the soft parts 32a and 32b may be properly formed in a portion coming in direct or indirect contact with the wearer.

The lock pin 40 is rotated around an axis in a state where the lock pin 40 is inserted in both the pin through hole 22d of the inner cup 20 and the pin through hole 35f of the outer cup 30 to thereby connect the inner cup 20 with the outer cup 30.

### Diaper 6

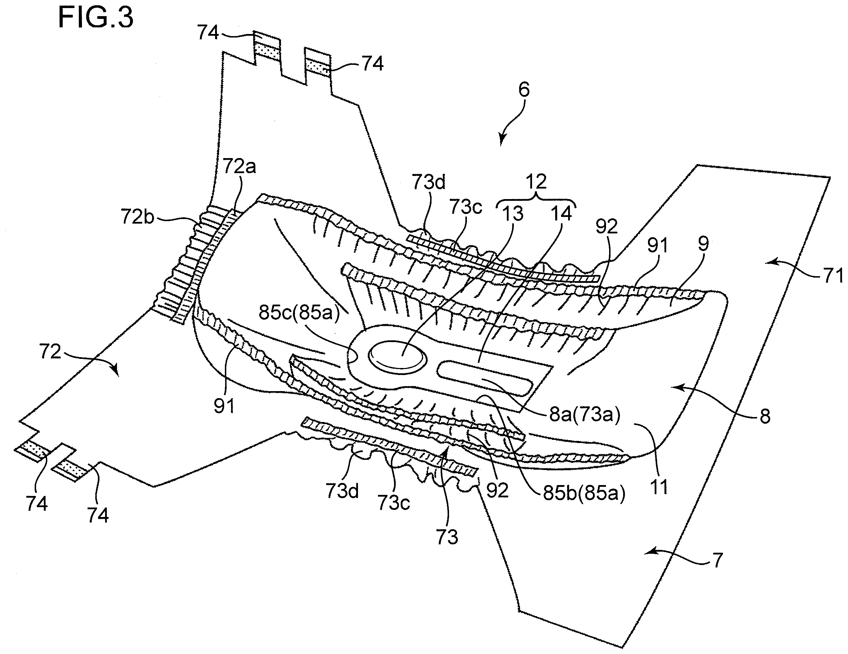

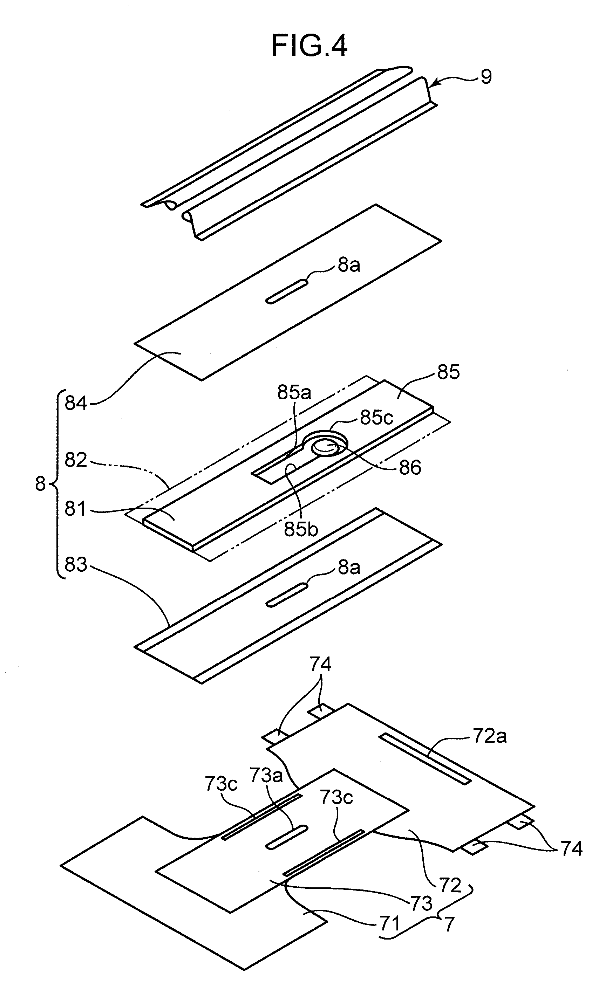

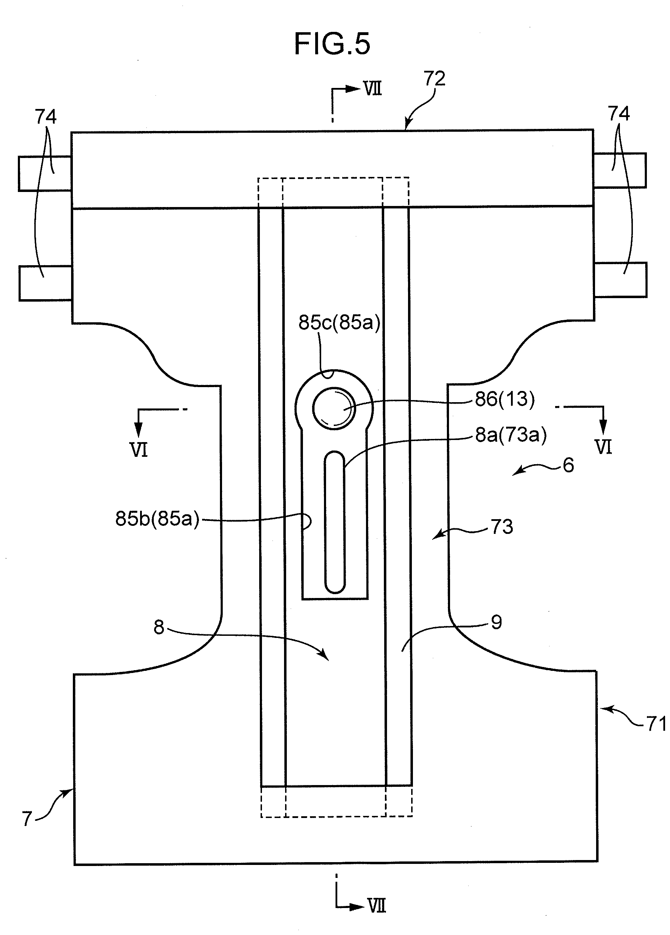

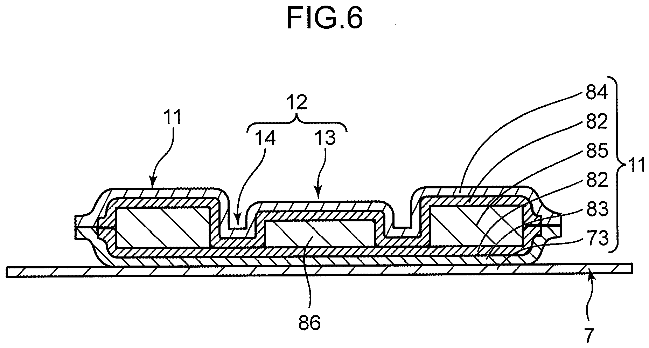

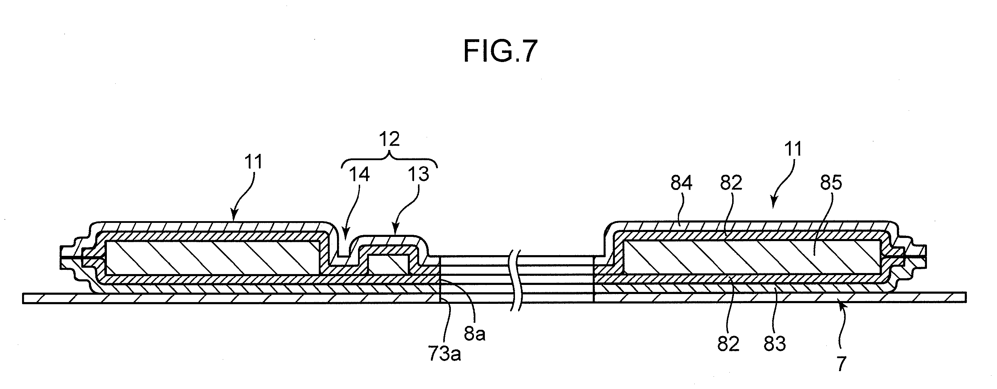

Hereinafter, the diaper 6 will be described with reference to FIG. 3 to FIG. 7. FIG. 3 is a perspective view of the diaper. FIG. 4 is an exploded perspective view of the diaper. FIG. 5 is a plan view showing a development of the diaper. FIG. 6 and FIG. 7 are a sectional view taken along a line VI-VI and a sectional view taken along a line VII-VII in FIG. 5, respectively.

The diaper 6 includes a diaper main body 7 and an absorbent 8 laid over the diaper main body 7. In this embodiment, the diaper 6 further includes standing flaps 9 that extend on widthwise both sides of the absorbent 8 in a longitudinal direction of the absorbent 8, and are made of a non-woven fabric. The standing flaps 9 are provided to keep excrement or the like from leaking from the widthwise both sides of the absorbent 8. The standing flaps 9 are respectively bonded with elastic members 91 in an expanded state along the longitudinal direction. This forms gathers 92 in the longitudinal direction of the standing flaps 9.

As is clearly shown in FIG. 4, the diaper main body 7 includes a front abdomen part 71 facing an abdomen of the wearer, a rear back part 72 facing a back of the wearer, a crotch part 73 arranged between respective widthwise central portions of the front abdomen part 71 and the rear back part 72, and fastening tapes 74 arranged on widthwise both end portions of the rear back part 72. The diaper main body 7 is attached to the wearer by fastening the front abdomen part 71 and the rear back part 72 by the fastening tapes 74 in a state where the crotch part 73 covers the inguinal region of the wearer and the front abdomen part 71 and the rear back part 72 surround a waist of the wearer. Thereby the diaper main body 7 can be held to the wearer.

The front abdomen part 71 and the rear back part 72 have a shape and structure that are symmetrical. The front abdomen part 71 and the rear back part 72 respectively include gathers formed by bonding a plurality of non-woven sheets to one another in a state where the plurality of non-woven sheets are layered to one another, and elastic members in an expanded state are bonded in proper positions between the non-woven sheets. FIG. 3 shows a gather 72b formed by an elastic member 72a bonded in a width direction of the rear back part 72, which is merely an example. The front abdomen part 71 also includes a similar gather formed along the width direction, which is not shown.

The crotch part 73 is made of a cover sheet that is liquid permeable or liquid impermeable. The crotch part 73 has a rectangular shape extending in a direction, and lengthwise both end portions bonded on the widthwise respective central portions of the front abdomen part 71 and the rear back part 72. The crotch part 73 includes a substantially lengthwise central portion formed with a unit passage hole 73a (an example of an opening) in. The unit passage hole 73a allows various components for connecting the inner cup 20 with the processing unit 5 to pass therethrough. In this embodiment, the unit passage hole 73a extends toward the front abdomen part 71 so as to allow the lock pin 40 to pass therethrough. Specifically, the unit passage hole 73a has a narrow shape extending in a longitudinal direction of the crotch part 73, and allows the drain tube part 23 of the inner cup 20 and the lock pin 40 to pass therethrough in an order in the longitudinal direction from the rear back part 72. This allows the unit passage hole 73a to cover positions corresponding to the drain tube part 23 of the inner cup 20 and the lock pin 40 when the diaper 6 is attached with the excrement cup 2.

In this embodiment, the unit passage hole 73a is configured to allow the lock pin 40 to pass therethrough, but a pin through hole allowing the lock pin 40 to pass therethrough may be separately formed. Further, as is clearly shown in FIG. 3, the crotch part 73 is bonded with elastic members 73c in an expanded state on the widthwise both edge portions thereof to form leg gathers 73d.

Hereinafter, the absorbent 8 will be described. As is clearly shown in FIG. 4, the absorbent 8 includes an absorbent core 81 extending in a direction, a wrapping sheet 82 wrapping the absorbent core 81, a back sheet 83 that is liquid impermeable and is arranged below the absorbent core 81 wrapped by the wrapping sheet 82, and a top sheet 84 that is liquid permeable and sandwiches the absorbent core 81 wrapped by the wrapping sheet 82 together with the back sheet 83 from above and below. The wrapping sheet 82 may be appropriately omitted.

The absorbent 8 is formed into a rectangular shape having a greater length than the crotch part 73 of the diaper main body 7. The absorbent 8 is formed with a unit passage hole 8a (an example of an opening) that is in communication with the unit passage hole 73a of the diaper main body 7 within an area of a below-described cup window 85a of the absorbent core 81 in a state where the unit passage hole 8a passes through the wrapping sheet 82, the back sheet 83, and the top sheet 84. In the same manner as the unit passage hole 73a, the unit passage hole 8a allows various components (the connection part) for connecting the inner cup 20 with the processing unit 5 to pass therethrough or allows the lock pin 40 to pass therethrough. Therefore, in the same manner as the unit passage hole 73a of the diaper main body 7, the unit passage hole 8a may be divided into separate holes. Further, it is not necessary that the unit passage holes 8a of the sheets 82 to 84 are formed at positions and in shapes completely overlapping with one another, but are formed so as to overlap vertically in at least one portions so as to perform the function. In this embodiment, the unit passage holes 8a of the sheets 82 to 84 are formed at positions and in shapes vertically overlapping with one another within an area of a below-described rectangular region 85b of the cup window 85a, and are formed at positions and in shapes overlapping the unit passage hole 73a of the diaper main body 7. In description of the sheets 82 to 84 hereinafter, the same reference numerals will be allotted in FIG. 4 to thereby omit description.

Specifically, the absorbent core 81 is formed by molding fluffs obtained by crushing and defiberizing rolled pulp, and is wrapped by the wrapping sheet 82 to have a certain firmness. The fluffs may be mixed with superabsorbent polymer.

The absorbent core 81 includes a main core 85 that is molded into a sheet shape extending from the front abdomen part 71 to the rear back part 72 and is formed with the cup window 85a in a lengthwise central portion and a sub core 86 that is arranged at a position in the cup window 85a close to the rear back part 72 and apart from the main core 85. In this embodiment, a basis weight of the main core 85 is set to be greater than a basis weight of the sub core 86, consequently the main core 85 has a thickness greater than a thickness of the sub core 86.

Specifically, the main core 85 is a main component of the first absorption part 11, and absorbs such liquid as excrement leaking from the cup main body 21 of the inner cup 20. In other words, the first absorption part 11 includes the main core 85, respective corresponding portions of a pair of upper and lower wrapping sheets 82 that are laid over and under the main core 85, the back sheet 83, and the top sheet 84, and a corresponding portion of the diaper main body 7.

The cup window 85a is formed so as to correspond to a portion sandwiched by the inner cup 20 and the outer cup 30 of the excrement cup 2, and opens across a specified longitudinal area in a widthwise central portion. The nipped sheet part 12 that is nipped by the inner cup 20 and the outer cup 30 includes the corresponding portions of the pair of upper and lower wrapping sheets 82, the back sheet 83, and the top sheet 84, the sub core 86, and the corresponding portion of the diaper main body 7, which are arranged in the cup window 85a.

Specifically, the cup window 85a corresponds to the shapes of the lower surface and the peripheral surface of the cup main body 21, and the outer surface and side surfaces of the urination guide part 22. In other words, the cup window 85a is formed so as to have a size and a shape equal to or covering the development of the inner wall surface (including the inner side surfaces and the bottom surface) of the fitting recess part 35c of the outer cup 30 accommodating the inner cup 20. The main core 85 has a basis weight of 400 to 600 g/m², and a thickness in a range of 5 to 10 mm. An outer edge of the cup window 85a is defined by junction of the upper and lower wrapping sheets 82 on the contour of the cup window 85a. In this embodiment, the cup window 85a has a shape of a keyhole including the rectangular region 85b close to the front abdomen part 71 and a circular region 85c continuing with the rectangular region 85b close to the rear back part 72. The rectangular region 85b is formed so as to conform to the shapes of the rear wall 22a and the side walls 22b of the urination guide part 22 of the inner cup 20. The circular region 85c is formed so as to conform to the shapes of the bottom wall 21b and the peripheral wall 21c of the cup main body 21 of the inner cup 20. The unit passage hole 8a of the absorbent 8 extends from a portion close to the rear back part 72 to a portion close to the front abdomen part 71 of the rectangular region 85b.

On the other hand, the sub core 86 is a main component of a second absorption part 13 to be described later, and absorbs liquid (e.g., excrement) leaking into a portion nipped by the inner cup 20 and the outer cup 30. Specifically, the second absorption part 13 includes the sub core 86, respective corresponding portions of the pair of upper and lower wrapping sheets 82 laid over and under the sub core 86, the back sheet 83, and the top sheet 84, and a corresponding portion of the diaper main body 7. A thin sheet part 14 includes a portion excluding the second absorption part of the nipped sheet part 12. Therefore, the thin sheet part 14 is thinner than both the first absorption part 11 and the second absorption part 13.

The sub core 86 has a smaller basis weight than the main core 85, and has a smaller thickness than the main core 85. Specifically, the sub core 86 has a basis weight of 50 to 400 g/m², and a thickness in a range of 0.5 to 5 mm. In a case where the sub core 86 is mixed with superabsorbent polymer, an amount added per unit weight to the sub core 86 may be regulated to be smaller than an amount added per unit weight to the main core 85. An outer edge of the sub core 86 is defined by junction of the upper and lower wrapping sheets 82. In this embodiment, the sub core 86 is arranged at a position facing the lower surface of the cup main body 21 of the inner cup 20, and has a shape conforming to the bottom wall 21b. Specifically, as shown in FIG. 5, the sub core 86 has substantially a circular shape in a plan view. A size of the sub core 86 is set to be a little smaller than the lower surface of the cup main body 21. Specifically, the shape of the sub core 86 is formed to be similar to the lower surface of the cup main body 21. In this case, a scale ratio is preferably set such that the sub core 86 has a size of 70% to 95% of the lower surface of the cup main body 21. The sub core 86 extends to a position overlapping the rectangular region 85b of the cup window 85a. Therefore, the sub core 86 extends to a position close to the unit passage hole 8a of the absorbent 8.

The wrapping sheet 82 is a liquid permeable sheet, covers the absorbent core 81, imparts the firmness to the absorbent core 81, and in a case where the absorbent core 81 contains superabsorbent polymer, suppresses dispersion of the polymer. In this embodiment, the wrapping sheets 82 includes a pair of upper and lower tissue paper sheets, and are joined to each other while interposing the absorbent core 81 therebetween. However, a single sheet of tissue paper may be folded and may sandwich the absorbent core 81 therebetween. The upper and lower wrapping sheets 82 are joined along the outer peripheral portion. The upper and lower wrapping sheets 82 are joined along the outer peripheral edge of the cup window 85a and the outer peripheral edge of the sub core 86. The joining portion may be either continuous or discontinuous. However, in the case where the superabsorbent polymer is mixed in the fluff of the absorbent core 81, the joining portion may be preferably continuous in view of suppressing dispersion of polymer.

The back sheet 83 is a liquid impermeable sheet, and prevents liquid caught by the absorbent core 81 from leaking outside. The back sheet 83 has a rectangular shape of a size larger than an outer dimension of the wrapping sheets 82 in which the absorbent core 81 is wrapped. On the other hand, the top sheet 84 is a liquid permeable sheet, and sandwiches the absorbent core 81 wrapped by the wrapping sheets 82 between the top sheet 84 and the back sheet 83. Similarly to the back sheet 83, the top sheet 84 also has a rectangular shape of a size larger than an outer dimension of the wrapping sheets 82 in which the absorbent core 81 is wrapped. The back sheet 83 and the top sheet 84 may be vertically joined together with the wrapping sheets 82 along the outer edge of the cup window 85a and an outer edge of the sub core 86 to define the outer edges in the same manner as the wrapping sheets 82.

The liquid impermeable sheet is preferably a polyethylene film or a nonwoven fabric having a water repellency and an air permeability. The liquid permeable sheet is preferably a nonwoven fabric or a mesh sheet having a liquid permeability. A plurality of the sheets may be laid one over another as long as it does not affect the functionalities of the liquid impermeability and the liquid permeability. A liquid permeable sheet may be laid over a liquid impermeable sheet.

The diaper 6 configured as described above is assembled in the following manner.

First, the absorbent 8 is formed. Specifically, a hot melt adhesive or the like is applied on one wrapping sheet 82, and a molded absorbent core 81 is laid thereover. The main core 85 and the sub core 86 are thus laid over the one wrapping sheet 82. Another wrapping sheet 82 is laid thereover. The pair of upper and lower wrapping sheets 82 are joined along the outer peripheral edge positioned outside the absorbent core 81 in the plan view, and the pair of upper and lower wrapping sheets 82 are joined along the outer edge of the cup window 85a and the outer edge of the sub core 86 to thereby wrap the absorbent core 81 with the wrapping sheets 82. The absorbent core 81 wrapped by the wrapping sheets 82 is nipped by the back sheet 83 and the top sheet 84, and the outer peripheral portions of the back sheet 83 and the top sheet 84 are joined. The absorbent 8 is thus formed. The unit passage hole 8a may be formed altogether after laminating the sheets 82 to 84, or may be formed in each of the sheets 82 to 84 and then the sheets 82 to 84 may be laminated in a state where the respective unit passage holes 8a are vertically aligned. Further, as described later, the absorbent 8 may be bonded to the diaper main body 7, and thereafter, the unit passage holes 8a and 73a may be formed together.

Next, the absorbent 8 and the standing flaps 9 are assembled with the diaper main body 7. First, the absorbent 8 is assembled with the diaper main body 7. Specifically, the absorbent 8 is adhered to the diaper main body 7 in a state where the absorbent 8 is disposed to extend from the front abdomen part 71 to the rear back part 72 via the crotch part 73. Further, the standing flaps 9 are adhered along widthwise both side edges of the absorbent 8 to thereby assemble the diaper 6.

Summarizing the diaper 6 according to the configuration of the present invention, the diaper 6 includes, in a portion corresponding to the crotch part 73 of the diaper main body 7, a sheet laminate including the pair of upper and lower wrapping sheets 82, the back sheet 83, the top sheet 84, and the cover sheet that constitutes the crotch part 73. A portion where the main core 85 is sandwiched by the sheet laminate (specifically, the pair of wrapping sheets 82) constitutes the first absorption part 11, and a portion where the sub core 86 is sandwiched by the sheet laminate constitutes the second absorption part 13. A portion of the sheet laminate provided outside the second absorption part 13 in the plan view and inside the first absorption part 11 in the plan view constitutes the thin sheet part 14. The nipped sheet part 12 is formed by the thin sheet part 14 and the second absorption part 13. Consequently, the diaper 6 includes the nipped sheet part 12 that is arranged so as to correspond to the cup window 85a of the main core 85 and is nipped by the inner cup 20 and the outer cup 30 and the first absorption part 11 that is arranged outside the nipped sheet part 12 in the plan view and includes the main core 85. The nipped sheet part 12 includes the thin sheet part 14 that is arranged so as to correspond to the outer peripheral portions of the rectangular region 85b and the circular region 85c of the cup window 85a and the second absorption part 13 that is arranged inside the thin sheet part 14 in the plan view and includes the sub core 86. The nipped sheet part 12 includes the unit passage holes 8a and 73a in an area corresponding to the rectangular region 85b. The second absorption part 13 is provided in the circular region 85c adjacent to the unit passage holes 8a and 73a. The first absorption part 11 absorbs liquid leaking from the inner cup 20, and liquid not absorbed by the first absorption part 11 is absorbed in the second absorption part 13 via the thin sheet part 14. The thin sheet part 14 is thinner than the first absorption part 11 by the thickness of the main core 85.

The diaper 6 configured as described above is attached with the excrement cup 2 as described below.

First, the diaper 6 is set in the outer cup 30. Specifically, the nipped sheet part 12 is arranged in the fitting recess part 35c of the outer cup 30 by utilizing the thin sheet part 14 of the diaper 6 as a guide mark. The nipped sheet part 12 is arranged in such a manner that the outer surface of the nipped sheet part 12 comes into tight contact with the inner wall surface (including the inner side surfaces and the bottom surface) of the fitting recess part 35c and the outer peripheral edge of the nipped sheet part 12 is folded at the first soft part 32a attached on the top portion of the outer peripheral wall 35b. In this state, the outer through hole 35d and the pin through hole 35f of the outer cup 30 face the unit passage holes 8a and 73a of the diaper 6.

As described above, since the nipped sheet part 12 includes the thin sheet part 14 that is arranged in the outer peripheral portion of the nipped sheet part 12 and is thinner than the first absorption part 11, the diaper 6 is easily positioned relative to the inner cup 20 and the outer cup 30 by utilizing the thin sheet part 14 as the guide mark when the diaper 6 is nipped by the cups 20 and 30.

Next, the inner cup 20 is attached to the outer cup 30 in a state where the diaper 6 is interposed therebetween. Specifically, the inner cup 20 is fitted in the fitting recess part 35c of the outer cup 30 by inserting the inner side engaging portion 21f of the inner cup 20 in the outer side engaging recess 35e of the outer cup 30. The outer cup 30 is thus fitted with the outside of the inner cup 20. The drain tube part 23 of the inner cup 20 is exposed outside the diaper 6 through the unit passage holes 8a and 73a of the diaper 6 and outside the outer cup 30 through the outer through hole 35d of the outer cup 30.

In this state, the pin through hole 22d of the inner cup 20 faces the pin through hole 35f and the unit passage holes 8a and 73a of the outer cup 30 and the diaper 6, respectively. Further, the thin sheet part 14 of the diaper 6 is interposed between the inner side engaging portion 21f and the outer side engaging recess 35e. In other words, the thin sheet part 14 includes a portion to be interposed between the inner side engaging portion 21f and the outer side engaging recess 35e. Thus, the excrement cup 2 merely requires an insertion and an engagement of the inner side engaging portion 21f in the outer side engaging recess 35e while interposing the thin sheet part 14 therebetween when the diaper 6 is nipped by the inner cup 20 and the outer cup 30, which improves the handleability. Additionally, since the inner side engaging portion 21f and the outer side engaging recess 35e are engaged with each other via the thin sheet part 14, the engaging state of the engaging portions 21f and 35e can be properly maintained. This can prevent an unexpected detachment of the outer cup 30 from the inner cup 20 which is likely to be caused by a disengagement of the inner side engaging portion 21f from the outer side engaging recess 35e due to the nipped sheet part 12.

Further, the thin sheet part 14 of the diaper 6 is placed in tight contact with both the outer peripheral surface of the inner cup 20 and the inner side surfaces of the fitting recess part 35c of the outer cup 30. In this manner, providing the thin sheet part 14 in the outer peripheral portion of the nipped sheet part 12 can reduce a dimensional error of a thickness of the outer peripheral portion of the nipped sheet part 12. This facilitates the management of a gap dimension between the inner cup 20 and the outer cup 30 that nip the thin sheet part 14 to thereby improve the tightness of the contact between the thin sheet part 14, the inner cup 20, and the outer cup 30, improving thus the sealability.

On the other hand, the second absorption part 13 of the diaper 6 is arranged between the lower surface of the cup main body 21 and the bottom surface of the fitting recess part 35c. Therefore, even in a case where the excrement or the like seeps between the inner cup 20 and the thin sheet part 14, the excrement can be absorbed by the second absorption part 13. Additionally, the second absorption part 13 is provided so as to face the lower surface of the cup main body 21 where the excrement such as feces and urine and rinse water concentrates, and is arranged at a lower position of the diaper 6 worn by the wearer. Therefore, even when the excrement seeps through the thin sheet part 14, the excrement is likely to concentrate in the second absorption part 13. Thus, the diaper 6 can efficiently absorb the excrement and the like by the second absorption part 13. Accordingly, the excrement which has seeped through the thin sheet part 14 can be effectively suppressed from leaking through the unit passage holes 8a and 73a of the diaper 6 or the outer through hole 35d of the outer cup 30. Consequently, the contamination of the outer cup 30 and the bedclothes with the excrement can be effectively suppressed.

Finally, the lock pin 40 is made to pass through the pin through holes 35f and 22d and the unit passage holes 8a and 73a, and the lock pin 40 is rotated to thereby mutually lock the inner cup 20 and the outer cup 30 in an attached state with the diaper 6 nipped therebetween. The excrement cup 2 can be thus attached to the diaper 6.

In order to put the diaper 6 attached with the excrement cup 2 on the wearer in the supine position, first, the rear back part 72 of the diaper 6 is laid below the back of the wearer. The second soft part 32b of the outer cup 30 is laid below the buttocks of the wearer, and the front surface of the inner cup 20 is disposed to face the inguinal region of the wearer. Further, the receiving space 21a of the cup main body 21 is disposed to face substantially upward, and the urination guide part 22 extends upward along the inguinal region of the wearer. Finally, the front abdomen part 71 of the diaper 6 is brought in contact with the abdomen of the wearer, and the rear back part 72 and the front abdomen part 71 are fastened by the fastening tapes 74 arranged on both end portions of the rear back part 72. Thus, the diaper 6 is put on the wearer P while holding the inner cup 20 to the wearer P in a state where the diaper 6 is nipped by the inner cup 20 and the outer cup 30. Therefore, a misplacement of the inner cup 20 relative to the diaper 6 can be suppressed, and the inner cup 20 can be put on the wearer P in a proper posture.

In this state, the rectangular region 85b of the diaper 6 is arranged along the urination guide part 22 extending upward, and the unit passage holes 8a and 73a of the diaper 6 are provided so as to correspond to the rectangular region 85b. This allows the unit passage holes 8a and 73a to be positioned above the lower surface of the cup main body 21. Therefore, it is difficult that the excrement which leaked in a periphery of the cup main body 21 leaks outside through the unit passage holes 8a and 73a.

The diaper 6 described above makes it possible to maintain a correct holding state of the inner cup 20 to the wearer P and suppress a leakage of the excrement leaking from the inner cup 20 through the unit passage holes 8a and 73a of the diaper 6, the outer through hole 35d or the pin through hole 35f of the outer cup 30 to thereby effectively suppress the contamination of the outer cup 30 and the bedclothes with the excrement.

In other words, since the diaper 6 includes the nipped sheet part 12 and the first absorption part 11 arranged outside the nipped sheet part 12 in the plan view, contaminants such as excrement and rinse water leaking from the inner cup 20 can be absorbed by the first absorption part 11, in particular, by the main core 85. Even in a case where the first absorption part 11 cannot absorb the entirety of the leaking excrement but a part of the excrement seeps between the inner cup 20 and the thin sheet part 14, the part of the excrement can be absorbed by the second absorption part 13. Therefore, the excrement seeped through the thin sheet part 14 can be effectively kept from leaking through the unit passage holes 8a and 73a of the diaper 6 or the outer through hole 35d of the outer cup 30. Therefore, the diaper 6 makes it possible to effectively prevent the contamination of the outer cup 30 and the bedclothes with the excrement.

The diaper 6 described above is merely an embodiment, and a specific configuration thereof may be properly modified. Hereinafter, modifications of this embodiment will be described.
(1) In the embodiment, the diaper 6 in the so-called tape form is described as a diaper 6. However, as mentioned above, the diaper 6 may be a wearable article in any form, e.g., a disposable diaper in the so-called pants form, a washable diaper capable of repeated use, or a combination of a diaper cover and a disposable water-absorbent sheet. For example, the diaper in the pants form may exclude the fastening tapes 74 of the diaper 6 of the embodiment and may be formed by superposing the widthwise both end portions of the front abdomen part 71 and the rear back part 72 to each other, and side-sealing them.
   Further, in the embodiment, the diaper main body 7 is bonded with the absorbent 8. However, a planar fastener and so on may be provided on the opposite surfaces of the diaper main body 7 and the absorbent 8, and the both 7 and 8 may be detachably attached with each other. In this case, the diaper main body 7 may be made of a washable fabric capable of repeated use.
(2) In the embodiment, the unit passage holes 8a and 73a of the diaper 6 are formed as a hole having a certain dimension. However, an opening of the wearable article may be a slit extending in a direction.
(3) In the embodiment, the diaper 6 is provided with the cover sheet serving as the crotch part 73 of the diaper main body 7 and the back sheet 83. However, the crotch part 73 may be omitted, and the back sheet 83 may be used as a substitution for the crotch part. The sheet structures thereof may be properly modified. Therefore, specific configurations of the nipped sheet part, the first absorption part, the second absorption part, and the thin sheet part may also be modified according to the sheet structures.
(4) In the embodiment, the second absorption part 13 is provided so as to face the lower surface of the cup main body 21. However, the position of the second absorption part is not particularly limited as long as the second absorption part is arranged inside the thin sheet part 14 in the plan view. For example, the second absorption part may be provided in the opening of the diaper 6, e.g., around the unit passage holes 8a and 73a. In this case, the second absorption part may be provided over an entire periphery of the opening, or may be provided discontinuously or in a partial area thereof. In a case where the second absorption part is provided in a partial area, and the opening is arranged so as to extend vertically in a state where the wearable article is worn by the wearer in the supine position, the second absorption part is preferably provided around a lower periphery of the opening for an efficient absorption of the excrement.
   Further, in the embodiment, the thin sheet part 14 is constituted by the sheet laminate (the pair of upper and lower wrapping sheets 82, the back sheet 83, the top sheet 84, and the cover sheet constituting the crotch part 73). However, the sheet structure may be properly modified, and a partial region of the sheet laminate may be laminated with an absorbent core having a thickness not greater than that of the sub core 86 to thereby impart the absorbency thereto. Consequently, the main core 85 and the sub core 86 may be partially connected by the absorbent core of the thin sheet part.
(5) In the embodiment, the inner cup 20 and the outer cup 30 are engaged with each other by the inner side engaging portion 21f and the outer side engaging recess 35e, but the engaging structure is not limited to a specific one. The inner cup 20 may be provided with an engaging recess and the outer cup 30 may be provided with an engaging protrusion, or the both may be provided with engaging claws to be engaged with each other. The specific structure may be properly modified.
(6) In the embodiment, the absorbent core 81 includes fluff as a component, but a transfer sheet may be additionally laminated therein. The transfer sheet rapidly disperses urine and the like and prevents the absorbed urine and the like from leaking from the top sheet 84.
(7) In the embodiment, the inner cup 20 may be further provided with a planar engaging member such as a planar fastener provided on at least one of a lower surface of the cup main body 21 and an outer surface of the urination guide part 22. The planar engaging member is caused to engage with the top sheet 84 made of a nonwoven fabric to suppress a misplacement of the inner cup 20 relative to the diaper 6. Further, in the embodiment, the diaper 6 is first set in the outer cup 30. However, in this case, the inner cup 20 and the diaper 6 may be provisionally assembled by the planar engaging member, and then the outer cup 30 may be attached thereto.
(8) The outer cup 30 of the embodiment may further include a sheet packing covering at least a lower edge portion of the outer through hole 35d in the inner accommodation part 35. The sheet gasket is formed by an elastic sheet made of rubber, urethane, and the like, and is elastically compressed on the drain tube part 23 in a state where the inner cup 20 is fitted in the inner accommodation part 35. The sheet gasket can thus keep the excrement that is not been absorbed by the second absorption part 13 and is leaked into the outer cup 30 through the unit passage holes 8a and 73a from leaking outside the outer cup 30.
(9) The automatic excrement processing apparatus 1 of the embodiment may additionally include a washing function of washing the inguinal region of the wearer after relieving or a drying function of drying the inguinal region after the washing.

It should be noted that the aforementioned specific embodiment principally includes the invention having the following configuration.

A wearable article which has solved the problem described above is a wearable article to be nipped by an inner member including a receiving part for receiving excrement of a wearer and a connection part extending from the receiving part to an outside and to be connected to a processing unit for processing the excrement received in the receiving part, and an outer member including a fitting recess part to be fitted to an outside of the inner member and a through hole in communication with the fitting recess part for allowing the connection part to pass, for holding the inner member to the wearer in a state that the inner member opposes an inguinal region of the wearer, and including a nipped sheet part to be nipped by the inner member and the outer member by the fitting of the inner member in the fitting recess part of the outer member, and having an opening for allowing the connection part to pass, and a first absorption part arranged outside of the nipped sheet part in a plan view for absorbing the excrement leaking from the receiving part, wherein the nipped sheet part includes a thin sheet part that is provided at least in an outer peripheral portion of the nipped sheet part and is thinner than the first absorption part, and a second absorption part that is provided inside the thin sheet part in the plan view.

The wearable article makes it possible to maintain a correct holding state of the inner member to the wearer and suppress a leakage of the excrement leaking from the inner member through an opening of the wearable article to thereby effectively suppress the contamination of the outer member and the bedclothes with the excrement.

Specifically, the wearable article is put on the wearer while holding the inner member to the wearer in a state where the wearable article is nipped by the inner member and the outer member. Thus, a misplacement of the inner member relative to the wearable article can be suppressed, and the inner member can be put on the wearer in a proper posture. Additionally, since the wearable article includes the nipped sheet part to be nipped by the inner member and the outer member and the first absorption part arranged outside the nipped sheet part in the plan view, excrement leaking from the inner member can be absorbed by the first absorption part arranged outside the nipped sheet part.

Further, the nipped sheet part includes a thin sheet part that is provided at least in an outer peripheral portion of the nipped sheet part and is thinner than the first absorption part. Therefore, the wearable article is easily positioned relative to the inner member and the outer member by utilizing the thin sheet part as the guide mark when the wearable article is nipped by the inner member and the outer member. Additionally, providing the thin sheet part in the outer peripheral portion of the nipped sheet part can reduce a dimensional error of a thickness of the outer peripheral portion of the nipped sheet part. This facilitates the management of a gap dimension between the inner member and the outer member that nipped the thin sheet part to thereby improve the tightness of the contact between the thin sheet part, the inner member, and the outer member, improving thus the sealability.

Additionally, the nipped sheet part includes the second absorption part provided inside the thin sheet part in the plan view. Thus, even in a case where the excrement seeps between the inner member and the thin sheet part, the excrement can be absorbed by the second absorption part. Accordingly, the excrement which has seeped through the thin sheet part can be suppressed from leaking through the opening or the through hole. Consequently, the contamination of the outer member and the bedclothes with the excrement can be effectively suppressed.

A specific configuration for attaching the inner member and the outer member that are used for the wearable article is not particularly limited. However, the inner member and the outer member are preferably attached by engaging with each other in at least a portion. This configuration merely requires an engagement between the respective engaging portions of the inner member and the outer member when the wearable article is nipped by the inner member and the outer member, which improves the handleability. In this case, the thin sheet part preferably includes a portion to be interposed between the respective engaging portions of the inner member and the outer member.

In this wearable article, the respective engaging portions of the inner member and the outer member are engaged with each other via the thin sheet part. Therefore, the engaging state of the engaging portions can be properly maintained. This can prevent an unexpected detachment of the outer member from the inner member which is likely to be caused by a disengagement between the engaging portions due to the nipped sheet part.

Specific configurations of the inner member and the outer member that are used for the wearable article are not particularly limited. However, it may be appreciated that the wearable article is nipped by the outer member and the inner member including the receiving part lying below buttocks of the wearer who is in a supine position, and a urination guide part that extends upward from the receiving part. In this case, the second absorption part is preferably arranged so as to face a lower surface of the receiving part.

The inner member can cause the receiving part to receive feces and cause the urination guide part to guide the urine to the receiving part. Since the excrement thus concentrates in the receiving part, the excrement is likely to leak from the receiving part, and the leaked excrement is liable to seep the lower surface of the receiving part. In the wearable article, even when the excrement seeps the lower surface of the receiving part through the thin sheet part, the excrement can be efficiently absorbed by the second absorption part. Accordingly, the leakage of the excrement through the opening and the through hole can be effectively prevented.

In this case, a region in which the opening in the wearable article is provided is not particularly limited. However, it may be appreciated that at least a part of the opening is provided in a region of the nipped sheet part to be disposed along the urination guide part.

The wearable article allows at least a part of the opening to be positioned above the lower surface of the receiving part in a state where the wearable article is worn by the wearer. Therefore, the excrement which has leaked in a periphery of the receiving part can be kept from leaking outside through the opening.

## Claims

1. A wearable article to be nipped by an inner member including a receiving part for receiving excrement of a wearer and a connection part extending from the receiving part to an outside and to be connected to a processing unit for processing the excrement received in the receiving part, and an outer member including a fitting recess part to be fitted to an outside of the inner member and a through hole in communication with the fitting recess part for allowing the connection part to pass, for holding the inner member to the wearer in a state that the inner member opposes an inguinal region of the wearer, the wearable article comprising:
a nipped sheet part to be nipped by the inner member and the outer member by the fitting of the inner member in the fitting recess part of the outer member, and having an opening for allowing the connection part to pass; and
a first absorption part arranged outside of the nipped sheet part in a plan view for absorbing the excrement leaking from the receiving part,
wherein the nipped sheet part includes:
a thin sheet part that is provided at least in an outer peripheral portion of the nipped sheet part and is thinner than the first absorption part, and
a second absorption part that is provided inside the thin sheet part in the plan view.

2. The wearable article according to claim 1, wherein
the wearable article is to be nipped by the inner member and the outer member which are engaged with each other in at least a portion,
the thin sheet part includes a portion to be interposed between the respective engaging portions of the inner member and the outer member.

3. The wearable article according to claim 1 or claim 2, wherein
the wearable article is to be nipped by the inner member and the outer member, the inner member including the receiving part lying below buttocks of the wearer who is in a supine position, and a urination guide part that extends upward from the receiving part, and
the second absorption part is arranged so as to face a lower surface of the receiving part.

4. The wearable article according to claim 3, wherein
at least a part of the opening is provided in a region of the nipped sheet part to be disposed along the urination guide part.
